# EUROPÄISCHE PATENTSCHRIFT

(11) **EP 2 349 987 B1**
(45) Veröffentlichungstag und Bekanntmachung des Hinweises auf die Patenterteilung: **14.11.2012**
(21) Anmeldenummer: 09783451.9
(22) Anmeldetag: 28.09.2009
(51) Int. Cl.: C07C 303/42, C07D 231/44, C07C 313/02, C07C 313/04, A01N 43/00

(54) **VERFAHREN ZUR HERSTELLUNG UND AUFREINIGUNG VON TRIFLUORMETHANSULFINSÄURE**
METHOD FOR PRODUCING AND PURIFYING TRIFLUOROMETHANESULFINIC ACID
PROCEDE DE PRODUCTION ET DE PURIFICATION D'ACIDE TRIFLUOROMETHANESULFINIQUE

(30) Priorität: 02.10.2008 EP 08165699
(43) Veröffentlichungstag der Anmeldung: 03.08.2011
(73) Patentinhaber: BASF SE, 67056 Ludwigshafen (DE); Merial Limited, Duluth, GA 30096-4640 (US)
(72) Erfinder: SUKOPP, Martin, 68165 Mannheim (DE); KORTE, Alexander, 67433 Neustadt (DE); FÜLSTER, Stefan, 67547 Worms (DE); KEIL, Michael, 67251 Freinsheim (DE); RACK, Michael, 69214 Eppelheim (DE)
(74) Vertreter: Niedenbrück, Matthias
(86) Internationale Anmeldenummer: PCT/EP2009/062484
(87) Internationale Veröffentlichungsnummer: WO 2010/037693

(56) Entgegenhaltungen:
- WO-A1-2008/055877
- WO-A1-2009/068533
- HARZDORF C ET AL: "Über Perfluoralkansulfinsäuren" BIOCHEMISTRY, AMERICAN CHEMICAL SOCIETY, EASTON, PA.; US, Nr. 1, 1. Januar 1973 (1973-01-01), Seiten 33-39, XP002491378 ISSN: 0006-2960

## Beschreibung

Die Erfindung betrifft ein Verfahren zur Aufreinigung von Trifluormethansulfinsäure, Verfahren zur Herstellung von aufgereinigter Trifluormethansulfinsäure und die Verwendung der aufgereinigten Trifluormethansulfinsäure zur Herstellung von trifluormethylsulfinylierten Pyrazolderivaten, insbesondere von Fipronil.

Trifluormethansulfinsäure (CF₃-SO₂H(I), im Folgenden TFMS) und ihre Salze finden Anwendung als Zwischenprodukte in der organischen Synthese, beispielsweise bei der Herstellung des Insektizids Fipronil (siehe z.B. US 5,618,945, WO 2008/055 880, WO 2008/055 879 und WO 2008/055 877).

Aus der Literatur sind bereits verschiedene Verfahren zur Herstellung der TFMS und ihrer Salze bekannt. Gemäß EP-A 165,136 werden bestimmte Metalle in Gegenwart von Schwefeldioxid in einem polar aprotischen Solvent bei einem Druck von mindestens 10⁸ Pa mit einem Trifluormethylhalogenid umgesetzt. In US 6,203,670 ist die Umsetzung von Trifluoressigsäurekaliumsalz mit SO₂ in einem polar aprotischen Lösungsmittel unter Einwirkung von Ultraschall beschrieben. C.P. Andrieux et al. (J. Am. Chem. Soc. 112 (1990) 786-791) beschreiben die reduktive elektrochemische Umsetzung von CF₃Br mit SO₂. US 6,399,815 beschreibt die Herstellung von Sulfinaten durch Reduktion von Sulfonylchloriden mit Sulfiten oder Hydrogensulfiten in der Gegenwart eines Hydrogenphosphats. In der WO 99/32439 ist die Synthese von fluorierten Sulfinaten durch Reaktion von fluorierten Sulfonylfluoriden mit Na₂SO₃/NaHCO₃ in einem wässrigen Medium in Gegenwart eines fluorierten Tensids oder eines organischen Cosolvens mit einem Siedepunkt unter 110°C beschrieben. C. Harzdorf et al. (Liebigs Ann. Chem. 1973, 33-39) beschreiben die Herstellung von TFMS durch Umsetzung von Trifluormethansulfonylfluorid mit Hydrazin in Methanol, Ansäuern mit HCl und anschließender fraktionierter Destillation.

Trotz der genannten Methoden besteht nach wie vor ein Bedarf für Verbesserungen, insbesondere, was die Herstellung der freien Säure in reiner Form angeht. Aufgabe der Erfindung ist es daher, ein verbessertes Verfahren zur Herstellung von TFMS in hoher Reinheit zur Verfügung zu stellen.

Es wurde gefunden, dass sich TFMS in hoher Reinheit durch azeotrope Destillation des Rohprodukts mit einem geeigneten organischen Lösungsmittel erhalten lässt.

Gegenstand der Erfindung ist daher ein Verfahren zur Aufreinigung von Trifluormethansulfinsäure (TFMS), wobei man TFMS als Rohprodukt in einem aromatischen

Lösungsmittel, das einen Siedepunkt < 170°C aufweist, vorlegt und unter einem verminderten Druck von 0,1 bis 500 mbar eine azeotrope Destillation durchführt.

Weiterhin Gegenstand der Erfindung ist ein Verfahren zur Herstellung von TFMS, wobei man
a) ein Salz der TFMS mit einer schwerflüchtigen Säure, die einen pKₛ-Wert von < -2 aufweist, umsetzt und
b) die erhaltene Roh-TFMS in Mischung mit einem aromatischen Lösungsmittel, das einen Siedepunkt < 170° C aufweist, einer azeotropen Destillation bei einem verminderten Druck im Bereich von 0,1 bis 500 mbar unterwirft.

Weiterhin Gegenstand der Erfindung ist ein Verfahren zur Herstellung von Fipronil, wobei die nach einem der obigen Verfahren erhaltene TFMS mit einem Pyrazolderivat der Formel (I) 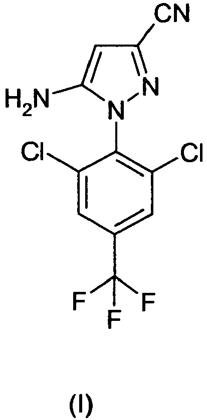 umgesetzt wird.

Das erfindungsgemäße Verfahren liefert TFMS in guter Ausbeute und hoher Reinheit, da das hauptsächliche Nebenprodukt der Reaktion, die höhersiedende Trifluormethansulfonsäure, kein Azeotrop bildet und mit dem Sumpf der Destillation einfach abgetrennt werden kann. Ein weiteres Nebenprodukt, die leichtflüchtige Trifluoressigsäure, kann ebenfalls destillativ als Vorfraktion abgetrennt werden. Durch die Verdünnung der Reaktionsmasse mittels Lösungsmittel werden Rührbarkeit und Wärmeübertragung verbessert, die Siedepunktserniedrigung durch das Azeotrop ermöglicht eine schonende Destillation.

Erfindungsgemäß wird eine Mischung aus TFMS-Rohprodukt und einem aromatischen Lösungsmittel mit einem Siedepunkt < 170°C unter einem verminderten Druck im Bereich von 0,1 bis 500 mbar azeotrop destilliert.

Alle Siedepunkte beziehen sich, soweit nicht anders angegeben, auf Normaldruck (1 bar).

Azeotrope Destillation bedeutet erfindungsgemäß, dass TFMS und aromatisches Lösungsmittel ein azeotropes Gemisch bilden, das heißt ein Gemisch, bei dem mit fortschreitender Destillation ein Punkt erreicht wird, an dem die Zusammensetzung von Flüssigphase(n) und Gasphase gleich wird, womit eine weitere destillative Auftrennung nicht mehr möglich ist. TFMS und das aromatische Lösungsmittel bilden üblicherweise ein Heteroazeotrop, das heißt die flüssige Phase ist bei der azeotropen Zusammensetzung instabil und zerfällt in zwei Phasen. Dies kann im Rahmen der Erfindung zur einfachen Abtrennung von TFMS mit einem Flüssig-Flüssig-Phasenscheider genutzt werden.

Als aromatisches Lösungsmittel eignen sich alle inerten aromatischen Lösungsmittel mit einem Siedepunkt von < 170°C, bevorzugt < 150°C, besonders bevorzugt < 140°C, die ein Azeotrop, vorzugsweise ein Azeotrop mit einem Siedepunktminimum, bilden. Inert bedeutet dabei im Sinne der Erfindung, dass unter den Bedingungen der Destillation keine oder eine die Ausbeute in nicht nachteiligem Umfang (unter 5%, bevorzugt unter 1 %) herabsetzende Reaktion zwischen TFSM und dem aromatischen Lösungsmittel stattfindet.

Geeignete aromatische Lösungsmittel sind beispielhaft in Tabelle 1 aufgeführt.

**Tabelle 1 Aromatische Lösungsmittel**

| **Lösungsmittel** | **Siedepunkt °C** |
|---|---|
| Benzol | 80 |
| Toluol | 110 |
| Xylol (Gemisch, o, m, p) | 140 |
| Chlorbenzol | 132 |
| Brombenzol | 156 |
| Fluorbenzol | 85 |
| Ethylbenzol | 136 |
| Anisol | 154 |
| Trifluormethylbenzol | 102 |
| Isopropylbenzol | 152 |
| Mesitylen | 165 |
| Chlortoluol (o, p, m) | 159-162 |

Um eine sinnvolle Destillation unter vermindertem Druck durchführen zu können, sollte der Siedepunkt des aromatischen Lösungsmittels vorzugsweise nicht unter 80°C liegen.

Bevorzugt sind Benzol, Fluorbenzol, Anisol, Trifluormethylbenzol, Toluol, Xylol, Ethylbenzol, Isopropylbenzol und Chlorbenzol. Besonders bevorzugt sind Benzol, Toluol, Xylol, Fluorbenzol, Trifluormethylbenzol, Ethylbenzol und Chlorbenzol. Insbesondere bevorzugt sind Toluol, Xylol, Ethylbenzol und Chlorbenzol. Natürlich können auch Mischungen von zwei oder mehr der genannten Lösungsmittel eingesetzt werden.

Das (Gewichts)Verhältnis von aromatischem Lösungsmittel zu TFMS kann in weiten Grenzen variieren und beträgt vorzugsweise von 1:100 bis 100:1, besonders bevorzugt von 1:3 bis 10:1, insbesondere von 1:1 bis 4:1.

Die Mischung aus Roh-TFMS und aromatischem Lösungsmittel kann durch Mischen der Roh-TFMS mit dem Lösungsmittel erfolgen. Bevorzugt ist es auch, ein Salz der TFMS in dem Lösungsmittel aufzunehmen und daraus durch Zugabe einer starken Säure TFMS freizusetzen.

Für die Destillation eignen sich alle bekannten Kolonnentypen, wie z.B. Füllkörperkolonnen, Bodenkolonnen, Packungskolonnen und Trennwandkolonnen. Bevorzugt sind Füllkörperkolonnen und Bodenkolonnen. In einer weiteren bevorzugten Ausführung des Verfahrens erfolgt die Destillation in einem Dünnschichtverdampfer, Fallfilmverdampfer oder Kurzwegverdampfer, einem der letztgenannten Apparate mit aufgesetzter Kolonne beliebiger Art, z.B. einer Packungskolonne, oder besonders bevorzugt, einem Reaktionsgefäß mit aufgesetzter Kolonne.

Die, gegebenenfalls fraktionierte, Destillation erfolgt je nach gewähltem Lösungsmittel im Allgemeinen in einem Temperaturbereich (Sumpf) von unter 110°C, da sonst die Gefahr der Zersetzung der TFMS besteht. Die Destillation wird unter vermindertem Druck, vorzugsweise in einem Bereich von 0,1 bis 500 mbar, bevorzugt von 1 bis 300 mbar, besonders bevorzugt von 10 bis 100 mbar durchgeführt, wobei der Siedepunkt des Azeotrops zu berücksichtigen ist.

Die erfindungsgemäß aufzureinigende TFMS kann nach beliebigen bekannten Verfahren hergestellt werden, beispielsweise nach einem der oben genannten Verfahren.

Wegen der geringen Stabilität von Trifluormethansulfinsäure wird diese Verbindung im Allgemeinen in Form eines Salzes gehandhabt und gelagert. Ein weiterer Gegenstand der Erfindung ist daher ein Verfahren zur Herstellung von TFMS, wobei TFMS durch eine starke Säure aus einem ihrer Salze freigesetzt wird und anschließend erfindungsgemäß durch azeotrope Destillation aufgereinigt werden.

Grundsätzlich können beliebige Salze der TFMS eingesetzt werden, bevorzugt sind Alkalimetalle, Erdalkalimetalle und Ammoniumsalze, besonders bevorzugt Alkalimetallsalze, insbesondere das Natrium- und Kaliumsalz.

Es können natürlich auch Mischungen aus zwei oder mehr verschiedenen Salzen eingesetzt werden.

Zur Freisetzung der TFMS aus dem Salz eignen sich schwerflüchtige Säuren mit einem pKₛ-Wert < -2. Schwerflüchtig im Sinne der Erfindung bedeutet, dass die Säure unter den Bedingungen der azeotropen Destillation nicht flüchtig ist und vorzugsweise einen Siedepunkt von mindestens 170°C bei Normaldruck aufweist. Beispiele für geeignete Säuren sind stark saure Ionenaustauscher, konzentrierte H₃PO₄ und H₂SO₄ insbesondere in konzentrierter Form (≥ 95 Gew.-% H₂SO₄), bevorzugt ist H₂SO₄.

Zur Freisetzung der TFMS ist mindestens ein Äquivalent Säure notwendig, bevorzugt werden 1 bis 10, besonders bevorzugt 1 bis 2, insbesondere 1 bis 1,5 äquivalente Säure eingesetzt.

Die Säure, insbesondere konzentrierte Schwefelsäure, kann auch als Lösungsmittel für das TFMS-Sulfinat eingesetzt werden. Bevorzugt ist es jedoch, das Salz in dem aromatischen Lösungsmittel zu suspendieren und die Säuren anschließend zuzugeben. Bevorzugt ist weiterhin eine kontinuierliche Zugabe unter Kühlung des Reaktionsgefäßes.

Weiterhin Gegenstand der Erfindung ist ein Verfahren zur Herstellung des Insektizids Fipronil (II), 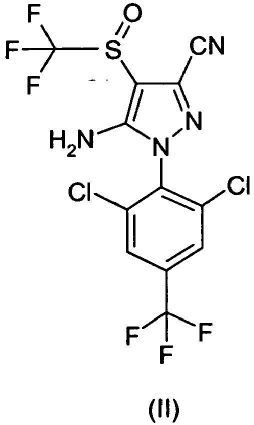 ohne weitere Aufreinigung der TFMS beispielsweise durch Redestillation, wobei man
a) ein Salz des TFMS mit einer schwerflüchtigen Säure, die einen pKₛ-Wert von < -2 aufweist, umsetzt und
b) die erhaltene Roh-TFMS im Gemisch mit einem aromatischen Lösungsmittel, das einen Siedepunkt < 170°C aufweist, unter vermindertem Druck azeotrop destilliert und
c) die erhaltene TFMS mit einem Pyrazolderivat der Formel (I) (siehe oben) umsetzt.

Verschiedene Verfahren zur Umsetzung des Pyrazolderivats (I) mit TFSM zu Fipronil (II) sind beispielsweise in US 5,618,945, WO 2008/055 880, WO 2008/055 879 und WO 2008/055 877 beschrieben.

Gemäß einer ersten Variante (i) (WO 2008/055 880) wird das Phenylpyrazolderivat (I) mit TFMS unter Zugabe eines Halogenierungsmittels in Gegenwart eines Säure/Amin-Komplexes umgesetzt, wobei das Amin ein sekundäres oder tertiäres Amin ist und als Säure HF, HCl, HBr, Hl oder ein Sulfonsäurederivat eingesetzt wird und wobei die Reaktionstemperatur zu keiner Zeit 39°C überschreitet.

Gemäß einer zweiten Variante (ii) (WO 2008/055 879) wird das Phenylpyrazolderivat (I) mit TFSM unter Zugabe eines Halogenierungsmittels in Gegenwart eines Säure/Amin-Komplexes umgesetzt, wobei das Amin ein cyclisches sekundäres Amin ist und als Säure ein Sulfonsäurederivat eingesetzt wird.

Gemäß einer dritten Variante (iii) (WO 2008/055 877) wird das Phenylpyrazolderivat (I) mit TFSM unter Zugabe eines Halogenierungsmittels in Gegenwart eines Säure/Amin-Komplexes umgesetzt, wobei das Amin ein tertiäres Amin ist und als Säure HF, HCl, HBr, Hl oder ein Sulfonsäurederivat eingesetzt wird.

In allen drei Varianten verläuft die Sulfinylierungsreaktion als ein zweistufiger Prozess ab, wobei im ersten Schritt eine Addition der CF₃S(O)-Gruppe an die Aminogruppe des Pyrazolrings stattfindet, gefolgt von einer Thia-Fries-Umlagerung zu Fipronil (II): 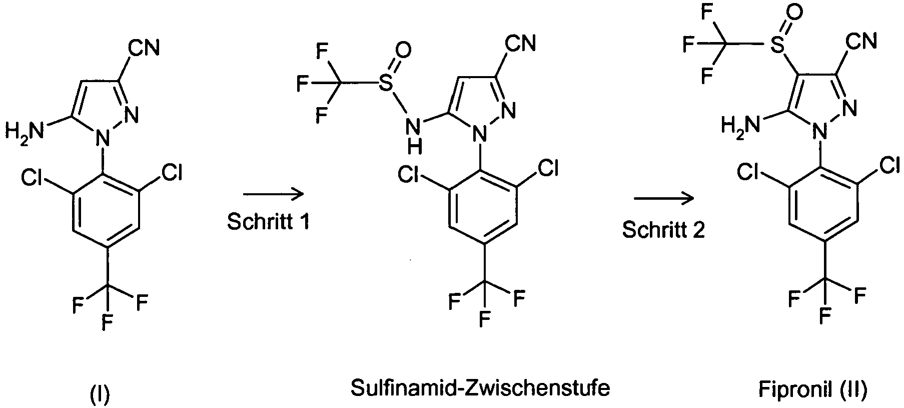 Einzelheiten der drei Varianten finden sich in den zitierten Schriften, auf die hiermit ausdrücklich Bezug genommen wird und deren Inhalt, soweit er die Umsetzung von TFSM betrifft, durch Zitat als Bestandteil dieser Beschreibung gilt.

In einer bevorzugten Ausführungsform des erfindungsgemäßen Verfahrens wird das erfindungsgemäß erhaltene TFSM-Destillat direkt ohne weitere Aufarbeitung zur Umsetzung mit dem Phenylpyrazolderivat (I) eingesetzt.

Erfindungsgemäß erhaltene TFSM kann neben der beschriebenen Verwendung weitere Anwendung als Zwischenprodukt in der organischen Synthese finden, beispielsweise in der pharmazeutischen Industrie oder zur Herstellung von Photoinitiatoren für die radikalische Polymerisierung von fluorierten Monomeren dienen (siehe z.B. WO 99/32439).

Die Erfindung wird durch die Beispiele näher erläutert, ohne sie dadurch einzuschränken.

### Beispiel 1: Azeotrope Destillation von Trifluormethansulfinsäure mit Ethylbenzol

In einen 750 mL Reaktor mit Rührer und Destillationsaufsatz wurden 92,7 g Kaliumtrifluormethansulfinat (0,50 mol, 92,5%ig) in 142 g Ethylbenzol suspendiert. Anschließend wurden bei 20 bis 30°C unter Kühlung 75,0 g konzentrierte Schwefelsäure (0,75 mol, 98%ig) innerhalb von 15 min zugetropft. Die dadurch freigesetzte Trifluormethansulfinsäure wurde innerhalb von 5 h bei einem Vakuum von 40 mbar und einer Kondensattemperatur von 46°C azeotrop abdestilliert. Die untere Phase des auf 5°C gekühlten Destillats mit 69,4 g bestand aus Trifluormethansulfinsäure und geringen Mengen Ethylbenzol. Die Oberphase, die hauptsächlich aus Ethylbenzol bestand, lief während der Destillation kontinuierlich in den Reaktor zurück. Die so gewonnene Trifluormethansulfinsäure (Ausbeute 95%, Reinheit 92%) kann ohne weitere Aufarbeitung in Folgereaktionen eingesetzt werden.

### Beispiel 2: Azeotrope Destillation von Trifluormethansulfinsäure mit Chlorbenzol

In einen 500 mL Vierhalskolben mit Rührer und Destillationsaufsatz wurden 50,0 g Natriumtrifluormethansulfinat (0,30 mol, 95,0%ig) in 144 g Monochlorbenzol (MCB) suspendiert. Anschließend wurden bei 20 bis 30°C unter Kühlung 48,5 g konzentrierte Schwefelsäure (0,48 mol, 97%ig) innerhalb von 10 min zugetropft. Die dadurch freigesetzte Trifluormethansulfinsäure wurde innerhalb von 6 h bei einem Vakuum von 36 mbar und einer Kondensattemperatur von 37°C azeotrop abdestilliert. Die untere Phase des auf -20°C gekühlten Destillats mit 40,1 g bestand aus Trifluormethansulfinsäure und geringen Mengen MCB. Die Oberphase, die hauptsächlich aus MCB bestand, lief während der Destillation kontinuierlich in den Reaktor zurück. Die so gewonnene Trifluormethansulfinsäure (Ausbeute 95%) kann ohne weiter Aufarbeitung in Folgereaktionen eingesetzt werden.

### Beispiel 3: Azeotrope Destillation von Trifluormethansulfinsäure mit Toluol

In einen 500 mL Vierhalskolben mit Rührer und Destillationsaufsatz wurden 50,0 g Natriumtrifluormethansulfinat (0,30 mol, 95,0%ig) in 112 g Toluol suspendiert. Anschließend wurden bei 20 bis 30°C unter Kühlung 46,1 g konzentrierte Schwefelsäure (0,46 mol, 97%ig) innerhalb von 10 min zugetropft. Die dadurch freigesetzte Trifluormethansulfinsäure wurde innerhalb von 9 h bei einem Vakuum von 50 bis 150 mbar und einer Kondensattemperatur von 31 bis 51°C azeotrop abdestilliert. Die untere Phase des auf -20°C gekühlten Destillats mit 34,2 g bestand aus Trifluormethansulfinsäure und geringen Mengen Toluol. Die Oberphase, die hauptsächlich aus Toluol bestand, lief während der Destillation kontinuierlich in den Reaktor zurück. Die so gewonnene Trifluormethansulfinsäure (Ausbeute 81%) kann ohne weitere Aufarbeitung in Folgereaktionen eingesetzt werden.

### Beispiel 4: Azeotrope Destillation von Trifluormethansulfinsäure mit Ethylbenzol in Anwesenheit von Trifluormethansulfonsäure

In einen 750 mL Reaktor mit Rührer und Destillationsaufsatz wurden 92,7 g Kaliumtrifluormethansulfinat (0,50 mol, 92,5%ig) in 142 g Ethylbenzol und 15,1 g Trifluormethansulfonsäure (0,1 mol, 99%ig) suspendiert. Anschließend wurden bei 20 bis 30°C unter Kühlung 75,0 g konzentrierte Schwefelsäure (0,75 mol, 98%ig) innerhalb von 15 min zugetropft. Die dadurch freigesetzte Trifluormethansulfinsäure wurde innerhalb von 5 h bei einem Vakuum von 37 mbar und einer Kondensattemperatur von 46°C azeotrop abdestilliert. Die untere Phase des auf 5°C gekühlten Destillats mit 57,8 g bestand laut ¹H- und ¹⁹F-NMR aus Trifluormethansulfinsäure, geringen Mengen Ethylbenzol und Spuren von Trifluormethansulfonsäure. Die Oberphase, die hauptsächlich aus Ethylbenzol bestand, lief während der Destillation kontinuierlich in den Reaktor zurück. Die so gewonnene Trifluormethansulfinsäure (Ausbeute 82%, Reinheit 95%) kann ohne weitere Aufarbeitung in Folgereaktionen eingesetzt werden.

### Beispiel 5: Azeotrope Destillation von Trifluormethansulfinsäure mit Ethylbenzol in Anwesenheit von Trifluoressigsäure

In einen 1000 mL Vierhalskolben mit Rührer und Destillationsaufsatz wurden ein Rohgemisch aus 50,8 g Kaliumtrifluormethansulfinat (0,29 mol, 98%ig) und 47,7 g Kaliumtrifluoracetat (0,31 mol, 99%ig) in 258 g Ethylbenzol suspendiert. Anschließend wurden bei 20 bis 30°C unter Kühlung 92,2 g konzentrierte Schwefelsäure (0,91 mol, 97%ig) innerhalb von 15 min zugetropft. Die dadurch freigesetzte Trifluoressigsäure wurde innerhalb von 20 min bei einem Vakuum von 40 mbar abdestilliert, während die ebenfalls freigesetzte Trifluormethansulfinsäure innerhalb von 3 h bei einem Vakuum von 40 mbar und einer Kondensattemperatur von 42 bis 46°C azeotrop abdestilliert wurde. Die untere Phase des auf 5°C gekühlten Destillats mit 30,0 g bestand aus Trifluormethansulfinsäure und geringen Mengen Ethylbenzol. Die Oberphase, die hauptsächlich aus Ethylbenzol bestand, lief während der Destillation kontinuierlich in den Reaktor zurück. Die so gewonnene Trifluormethansulfinsäure (Ausbeute 73%, Reinheit 95%) kann ohne weitere Aufarbeitung in Folgereaktionen eingesetzt werden.

### Beispiel 6: Verwendung erfindungsgemäß hergestellter Trifluormethansulfinsäure zur Synthese von Fipronil

In einen 500 mL Doppelmantelreaktor mit Rührer, Stromstörer und Kühler wurden unter Stickstoffatmosphäre 103 g Ethylbenzol, 6,3 g Dimethylisopropylaminhydrochlorid (0,050 mol, 99%ig) und 15,5 g Kaliumchlorid (0,208 mmol) vorgelegt. Anschließend wurden 31,5 g Trifluormethansulfinsäure (0,223 mol, 95,0%ig), 17,9 g Dimethylisopropylamin (0,203 mol, 99%ig) und 24,2 g Thionylchlorid (203 mmol, 99,7%ig) unter Kühlung bei 0°C zudosiert. Nach anschließender Zugabe von 54,8 g 5-Amino-3-cyano-1-(2,6-dichlor-4-trifluormethyphenyl)pyrazol wurde das Reaktionsgemisch für 1 h bei 0°C gerührt, im Anschluss über 45 min auf 35°C erhitzt und für 10 h bei 35°C nachgerührt. Nach Quenchen der Reaktion mit Natronlauge und Extraktion mit Ethylacetat und Ethylbenzol ergab sich eine nicht-isolierte Ausbeute an Fipronil von 80% in der Rohlösung (Bestimmung über quantitative HPLC).

## Patentansprüche

1. Verfahren zur Aufreinigung von Trifluormethansulfinsäure (TFMS), wobei man eine Mischung enthaltend Roh-TFMS und ein inertes aromatisches Lösungsmittel mit einem Siedepunkt < 170°C unter vermindertem Druck azeotrop destilliert.

2. Verfahren nach Anspruch 1, wobei das aromatische Lösungsmittel aus der Gruppe Benzol, Fluorbenzol, Anisol, Trifluormethylbenzol, Toluol, Xylol, Ethylbenzol, Isopropylbenzol und Chlorbenzol gewählt ist.

3. Verfahren nach Anspruch 1 oder 2, wobei das Gewichtsverhältnis von aromatischem Lösungsmittel zu TFMS 1:100 bis 100:1 beträgt.

4. Verfahren nach einem der Ansprüche 1 bis 3, wobei gegebenenfalls vorhandene Trifluoressigsäure im Vorlauf abgetrennt wird.

5. Verfahren nach einem der Ansprüche 1 bis 4, wobei gegebenenfalls vorhandene Trifluormethansulfonsäure mit dem Sumpf der Destillation abgetrennt wird.

6. Verfahren zur Herstellung von TFMS, wobei man
a) ein Salz der TFMS mit einer schwerflüchtigen Säure, die einen pKₛ-Wert von < -2 aufweist, umsetzt und
b) die erhaltene Roh-TFMS im Gemisch mit einem aromatischen Lösungsmittel, das einen Siedepunkt < 170°C aufweist, unter vermindertem Druck gemäß einem der Ansprüche 1 bis 5 azeotrop destilliert.

7. Verfahren gemäß Anspruch 6, wobei die schwerflüchtige Säure konzentrierte H₂SO₄ ist.

8. Verfahren zur Herstellung von Fipronil, wobei man
a) ein Salz des TFMS mit einer schwerflüchtigen Säure, die einen pKₛ-Wert von < -2 aufweist, umsetzt und
b) die erhaltene Roh-TFMS im Gemisch mit einem aromatischen Lösungsmittel, das einen Siedepunkt < 170°C aufweist, unter vermindertem Druck gemäß einer der Ansprüche 1 bis 5 azeotrop destilliert und
c) die erhaltene TFMS mit einem Pyrazolderivat der Formel (I) 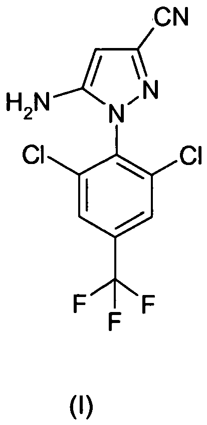 umsetzt.

9. Verfahren gemäß Anspruch 8, wobei die schwerflüchtige Säure H₂SO₄ ist.

10. Verfahren gemäß Anspruch 8 oder 9, wobei die in Schritt b) erhaltene TFMS ohne weiteren Reinigungsschritt direkt umgesetzt wird.

## Claims

1. A process for purifying trifluoromethanesulfinic acid (TFMS) by azeotropically distilling a mixture comprising crude TFMS and an inert aromatic solvent having a boiling point of < 170°C under reduced pressure.

2. The process according to claim 1, wherein the aromatic solvent is selected from the group of benzene, fluorobenzene, anisole, trifluoromethylbenzene, toluene, xylene, ethylbenzene, isopropylbenzene and chlorobenzene.

3. The process according to claim 1 or 2, wherein the weight ratio of aromatic solvent to TFMS is from 1:100 to 100:1.

4. The process according to any one of claims 1 to 3, wherein any trifluoroacetic acid present is removed in the first runnings.

5. The process according to any one of claims 1 to 4, wherein any trifluoromethanesulfonic acid present is removed with the bottoms of the distillation.

6. A process for preparing TFMS by
a) reacting a salt of TFMS with a nonvolatile acid which has a pKₛ of < -2, and
b) azeotropically distilling the resulting crude TFMS in a mixture with an aromatic solvent which has a boiling point of < 170°C under reduced pressure according to any one of claims 1 to 5.

7. The process according to claim 6, wherein the nonvolatile acid is concentrated H₂SO₄.

8. A process for preparing fipronil by
a) reacting a salt of TFMS with a nonvolatile acid which has a pKₛ of < -2, and
b) azeotropically distilling the resulting crude TFMS in a mixture with an aromatic solvent which has a boiling point of < 170°C under reduced pressure according to any one of claims 1 to 5 and
c) reacting the resulting TFMS with a pyrazole derivative of the formula (I)

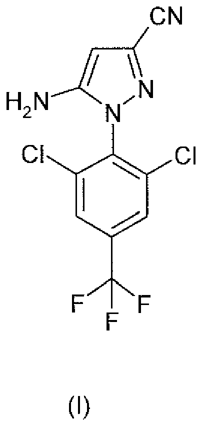

9. The process according to claim 8, wherein the nonvolatile acid is H₂SO₄.

10. The process according to claim 8 or 9, wherein the TFMS obtained in step b) is reacted directly without a further purification step.

## Revendications

1. Procédé pour la purification d'acide trifluorométhanesulfinique (TFMS), dans lequel on soumet à une distillation azéotropique sous pression réduite un mélange contenant du TFMS brut et un solvant aromatique inerte ayant un point d'ébullition < 170 °C.

2. Procédé selon la revendication 1, dans lequel le solvant aromatique est choisi dans le groupe constitué par le benzène, le fluorobenzène, l'anisole, le trifluorométhylbenzène, le toluène, le xylène, l'éthylbenzène, l'isopropylbenzène et le chlorobenzène.

3. Procédé selon la revendication 1 ou 2, dans lequel le rapport pondéral du solvant aromatique au TFMS vaut de 1:100 à 100:1.

4. Procédé selon l'une quelconque des revendications 1 à 3, dans lequel l'acide trifluoroacétique éventuellement présent est séparé dans la fraction de tête.

5. Procédé selon l'une quelconque des revendications 1 à 4, dans lequel l'acide trifluoro-méthanesulfonique éventuellement présent est séparé avec le résidu de la distillation.

6. Procédé pour la préparation de TFMS, dans lequel on
a) fait réagir un sel du TFMS avec un acide à faible volatilité, qui présente un indice pKₐ de < -2 et
b) soumet à une distillation azéotropique sous pression réduite, selon l'une quelconque des revendications 1 à 5, le TFMS brut obtenu, en mélange avec un solvant aromatique qui présente un point d'ébullition < 170 °C.

7. Procédé selon la revendication 6, dans lequel l'acide à faible volatilité est H₂SO₄ concentré.

8. Procédé pour la préparation de fipronil, dans lequel on
a) fait réagir un sel du TFMS avec un acide à faible volatilité, qui présente un indice pKₐ de < -2 et
b) soumet à une distillation azéotropique sous pression réduite, selon l'une quelconque des revendications 1 à 5, le TFMS brut obtenu, en mélange avec un solvant aromatique qui présente un point d'ébullition < 170 °C et
c) fait réagir le TFMS obtenu avec un dérivé de pyrazole de formule (I)

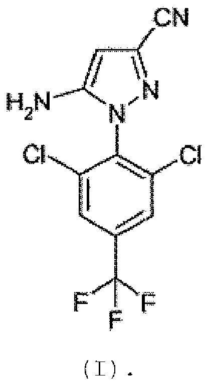

9. Procédé selon la revendication 8, dans lequel l'acide à faible volatilité est H₂SO₄.

10. Procédé selon la revendication 8 ou 9, dans lequel le TFMS obtenu dans l'étape b) est mis en réaction directement sans autre étape de purification.
